(19) Europäisches Patentamt
European Patent Office
Office européen des brevets

(11) **EP 3 995 522 A1**

(12) **EUROPEAN PATENT APPLICATION**

(43) Date of publication:
**11.05.2022 Bulletin 2022/19**

(21) Application number: **20206269.1**

(22) Date of filing: **06.11.2020**

(51) International Patent Classification (IPC):
**C08G 18/32** (2006.01)    **C08G 18/48** (2006.01)
**C08G 18/73** (2006.01)    **C08G 18/30** (2006.01)
**A61F 13/00** (2006.01)    **A61L 15/60** (2006.01)
**C08G 18/16** (2006.01)

(52) Cooperative Patent Classification (CPC):
(C-Sets available)
**C08G 18/3206; A61F 13/00017; A61F 13/00038;
A61L 15/60; C08G 18/168; C08G 18/302;
C08G 18/485; C08G 18/73;** C08G 2110/0083;
C08G 2110/0091        (Cont.)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
• **Covestro LLC
Pittsburgh, PA 15205-9741 (US)**

• **Covestro Deutschland AG
51373 Leverkusen (DE)**

(72) Inventor: **The designation of the inventor has not
yet been filed**

(74) Representative: **Levpat
c/o Covestro AG
Gebäude 4825
51365 Leverkusen (DE)**

(54) **METHOD FOR PRODUCING POLYURETHANE FOAMS OR HYDROGELS BY EMPLOYING DIOL CONTAINING FORMULATIONS**

(57)      The invention provides a process for producing polyurethane foams or hydrogels, in which compositions comprising

A) isocyanate-functional prepolymers obtainable by the reaction of

A1) low molecular weight diisocyanates of molar mass from 140 to 278 g/mol, with A2) polyalkylene oxides having an OH functionality of two or more,

A3) more than 1% by weight of $C_2$ to $C_{12}$ diols based on the total amount of the isocyanate-reactive components A2) to A4) wherein component A3) differs from component A2), A4) optionally further isocyanate-reactive components differing from A2) or A3);

B) water or a nonaqueous isocyanate-reactive component in an amount of at least 2% by weight, based on the total weight of the composition;

C) optionally polyisocyanates obtainable by reaction of at least two low molecular, preferably aliphatic diisocyanates, wherein the diisocyanates having a molar mass of 140 to 278 g/mol,

D) optionally catalysts;

E) optionally salts of weak acids, the corresponding free acids of which have a pKA in water at 25°C of $\geq$ 3.0 and $\leq$ 14.0;

F) optionally surfactants; and

G) optionally mono- or polyhydric alcohols or polyols;

H) optionally hydrophilic polyisocyanates obtainable by reaction of

HI) low molecular weight diisocyanates of molar mass from 140 to 278 g/mol and/or polyisocyanates preparable therefrom and having an average isocyanate functionality of 2 to 6 with

H2) monohydroxyfunctional polyalkylene oxides of OH number from 10 to 250 and of oxyethylene unit content from 50 to 100 mol%, based on the total amount of the oxyalkylene groups present,

are provided, optionally foamed and cured wherein the isocyanate containing components, especially components A), C) and H), do not exceed a residual diisocyanate content of 8% by weight, based on the total amount of the polyurethane foam or hydrogel. These foams or hydrogels are used in wound dressings, cosmetic articles or incontinence products.

EP 3 995 522 A1

(52) Cooperative Patent Classification (CPC): (Cont.)

C-Sets
**A61L 15/60**

**Description**

The invention provides a process for producing polyurethane foams or polyurethane hydrogels, in which compositions comprising A) isocyanate-functional prepolymers obtainable by the reaction of A1) low molecular weight diisocyanates of molar mass from 140 to 278 g/mol, with A2) polyalkylene oxides having an OH functionality of two or more and A3) more than 1% by weight $C_2$ to $C_{12}$ diols based on the total amount of the isocyanate-reactive components A2) to A4) wherein component A3) differs from those of component A2) and optionally A4) further isocyanate-reactive components differing from A2) and A3); B) water or a nonaqueous isocyanate-reactive component; C) optionally polyisocyanates composed of at least two low molecular weight, preferably aliphatic diisocyanates; D) optionally catalysts: E) optionally salts of weak acids, the corresponding free acids of which have a pKA in water at 25°C of $\geq 3.0$ and $\leq 14.0$; F) optionally surfactants; G) optionally mono- or polyhydric alcohols or polyols; H) optionally hydrophilic polyisocyanates, are provided, and also the foams and hydrogels thus produced and the uses thereof.

Water absorbing polyurethanes are useful for many applications such as foam or hydrogel based wound dressings, incontinence pads or hydrogel contact lenses. Foams are highly porous materials with low density. In the case of wound dressing foams, densities are often around 100 g/L. With increasing density the material may be called a porous hydrogel until it is a pure and compact hydrogel without any visible pores. There is no sharp boundary between the two extremes. Regardless of terminology, regarding foams and hydrogels, important mechanical properties, such as tensile strength at break, drastically decline upon swelling of the foams or hydrogels when coming into contact with fluids. Especially, for the application in wound dressings, water absorbing polyurethanes in the form of foams or hydrogels that retain high wet-tensile-strength-at-break ($\sigma_{B,wet}$) despite good water absorption are desirable.

Since the density of the material, like foams or hydrogels, has also a strong influence on the mechanical properties it is not easy to say which basic materials would directly reach the goal of retaining wet high wet-tensile-strength-at-break ($\sigma_{B,wet}$) and good water absorption. Solely, comparing absolute values of wet-tensile-strength-at-break ($\sigma_{B,wet}$) for the different materials like low density foams, high density foams and hydrogels, does not allow for assessing the mechanical performance of the polymer material they are made of. However, dividing $\sigma_{B,wet}$ by density (D) delivers a value that accounts for density differences and therefore normalizes the value of wet-tensile-strength-at-break ($\sigma_{B,wet}$) with respect to the density. However, it is not desirable to increase wet-tensile-strength-at-break ($\sigma_{B,wet}$) by simply reducing water absorption of the material because water absorption is an essential feature for the application in wound dressings and incontinence pads of these polymeric materials. Consequently, a material at low density with high water absorption that displays high $\sigma_{B,wet}$ is desirable.

When absorbing water, the material swells. Here, swelling was described as an expansion factor S in one dimension, i.e., the increase of a cross-section of a sample can be described by a factor $S^2$. Since tensile stress depends on the tensile force per cross-sectional area and water does not contribute to the tensile stress it is expected that wet-tensile-strength-at-break ($\sigma_{B,wet}$) drops by at least the factor $S^2$. Based on these considerations the term ($\sigma_{B,wet}$/D)$\cdot S^2$ is useful to assess the performance of the material of the foam or hydrogel in the wet state independent from density and extent of swelling.

Foams with good wet-tensile-strength-at-break ($\sigma_{B,wet}$) are already known. For instance, a foam from prepolymer example 4 of US2006142529 was synthesized (comparative example 17 in this document) and used to obtain a foam with an excellent ($\sigma_{B,wet}$/D)$\cdot S^2$ value of 0.61. However, all foams in US2006142529 were synthesized from prepolymers that were not distilled after synthesis, hence, containing more than at least 8% residual diisocyanate. Due to the low vapor pressure of diisocyanate monomers foam or hydrogel manufacturers require an exhaustion system for safe manufacturing of foams and hydrogels.

Another disadvantage of prepolymers in US2006142529 is that they are based on aromatic isocyanates. Furthermore, for many manufacturing processes it is desirable to use prepolymers with viscosities lower than 25000 mPas, preferably lower than 10000 mPas. Many aromatic prepolymers do not meet this requirement. Moreover, removing the residual diisocyanate by distillation will lead to a further drastic increase of viscosity. An additional disadvantage of aromatic prepolymers is that foams produced from aromatic prepolymers are inherently yellowing.

All foams in US2006142529 are based on aromatic isocyanates and contain > 8% monomeric diisocyanates. Similar foams are known from US3903232, US4137200 and US5849850.

Prior art documents EP2143744, EP2470580 EP2632501 and WO19137879, describe foams produced from aliphatic isocyanate prepolymers where the residual diisocyanate was removed via distillation. However, good wet strength performance was not mentioned in any of them.

All of the aforementioned aromatic or aliphatic prepolymer formulations were based on isocyanates that were reacted either solely with polyalkylene oxides or in combination with low molecular weight polyols that have functionalities greater than 2. The non-inventive examples 13 to 17 show that foams made of these components have poor ($\sigma_{B,wet}$/D)$\cdot S^2$ values because no diisocyanate monomers and/or inventive diols are present.

An aim that has not been achieved to date in the production of polyurethane foams or hydrogels, especially in the use thereof in medical applications, is to provide a process that is safe and allows a good processability, like an easy handling of all components and a fast processing of the basic materials and allows the manufacturing of foams or hydrogels that combine high water absorption with high wet-tensile-strength-at-break ($\sigma_{B,wet}$).

One aim of the present invention was that of at least partly overcoming at least one disadvantage of the prior art.

Furthermore, it was a goal to find favourable prepolymers that are easily pumpable and show good properties when mixed with components comprising water.

Therefore, it was a goal to produce foams or hydrogels by a fast and safe process which retain their hydrophilicity and ability to swell, especially as indicated by an edge length expansion factor S of at least 1.05. Furthermore, it was a goal that the wet elongation at break of the produced foams or hydrogels should be higher than 50%. In the particular case of foams with densities lower than 200 g/L the absolute wet-tensile-strength-at-break ($\sigma_{B,wet}$) should be at least 19 kPa.

A further goal of the invention was to provide non-yellowing foams or hydrogels, especially for the production of wound dressings.

Furthermore, it was a goal of the invention to provide a process for the production of polyurethane foams or hydrogels from prepolymer formulations that may be handled without health risks.

A further goal of the invention was to provide foams with good wet strength performance as indicated by ($\sigma_{B,wet}$/D)·$S^2$ values of at least 0.30, especially together with the properties and production features as mentioned above.

Furthermore, a high performing material which is characterized by a high ($\sigma_{B,wet}$) despite low density and high water absorption was a goal of the invention.

It has been found that, surprisingly, the combination of features of the subject-matter of Claim 1 was able to solve at least one of the mentioned problems or have reached the mentioned aims.

The invention firstly relates to a process for producing polyurethane foams or hydrogels, in which compositions comprising

> A) isocyanate-functional prepolymers obtainable by the reaction of
>
>> A1) low molecular weight diisocyanates of molar mass from 140 to 278 g/mol, with
>>
>> A2) polyalkylene oxides having an OH functionality of two or more, preferably within a range from 2 to 6, preferably within a range from 2 to 5, or preferably 2 to 4,
>>
>> A3) more than 1% by weight of $C_2$ to $C_{12}$ diols based on the total amount of the isocyanate-reactive components A2) to A4) wherein component A3) differs from component A2),
>>
>> A4) optionally further isocyanate-reactive components differing from A2) and A3);
>
> B) water or a nonaqueous isocyanate-reactive component in an amount of at least 2% by weight, preferably of at least 5% by weight, or preferably of at least 10% by weight, or preferably within a range from 2% to 40% by weight, or preferably within a range from 5% to 30% by weight, or preferably within a range from 10% to 25% by weight, based on the total weight of the composition;
>
> C) optionally polyisocyanates obtainable by reaction of at least two low molecular, preferably aliphatic diisocyanates, wherein the diisocyanates having a molar mass of 140 to 278 g/mol;
>
> D) optionally catalysts;
>
> E) optionally salts of weak acids, the corresponding free acids of which have a pKA in water at 25°C of ≥ 3.0 and ≤ 14.0;
>
> F) optionally surfactants; and
>
> G) optionally mono- or polyhydric alcohols or polyols;
>
> H) optionally hydrophilic polyisocyanates obtainable by reaction of
>
>> H1) low molecular weight diisocyanates of molar mass from 140 to 278 g/mol and/or polyisocyanates preparable therefrom and having an average isocyanate functionality of 2 to 6 with
>>
>> H2) monohydroxyfunctional polyalkylene oxides of OH number from 10 to 250 and of oxyethylene unit content from 50 to 100 mol%, based on the total amount of the oxyalkylene groups present,

are provided, eventually foamed and cured, wherein the isocyanate containing components, especially components A), C) and H), do not exceed a residual diisocyanate content of 8% by weight, based on the total weight of the polyurethane foam or hydrogel.

Preferably, the prepolymers A) have a residual diisocyanate content of below 5% by weight, more preferably below 3% by weight, especially preferably below 2%, even more preferably below 0.5% by weight, most preferably below 0.1% by weight based on the total weight of the prepolymer.

Preferably, the components A), C) and H) have a residual monomer content of below 5% by weight, more preferably below 3% by weight, especially preferably below 2%, even more preferably below 0.5% by weight, most preferably below 0.1% by weight, based on the total weight of the components A), C) and H).

The residual diisocyanate content according to the invention means the content of diisocyanates which have a molar mass from 140 to 278 g/mol.

In the preparation of the isocyanate-functional prepolymers A), the amounts of polyalkylene oxides A2), the $C_2$ to $C_{12}$ diols A3), A4) and the low molecular weight, preferably aliphatic diisocyanates A1) are preferably adjusted such that, for every 1 mol of OH groups of the combined components A2), A3) and A4), there are 1.1 to 20 mol, preferably 1.5 to 10 mol and more preferably 2 to 5 mol of NCO groups of the low molecular weight, preferably aliphatic diisocyanate A1).

In the preparation of the isocyanate-functional prepolymers A), the amounts of polyalkylene oxides A2), the $C_2$ to $C_{12}$ diols A3), the isocyanate reactive components A4) and the low molecular weight, preferably aliphatic diisocyanates A1) are preferably adjusted such that, for every 1 mol of isocyanate reactive groups of the combined components A2), A3), and A4), there are 1.1 to 20 mol, preferably 1.5 to 10 mol and more preferably 2 to 5 mol of NCO groups of the low molecular weight, preferably aliphatic diisocyanate A1).

The reaction can be effected in the presence of urethanization catalysts such as tin compounds, zinc compounds, amines, guanidines or amidines, or in the presence of allophanatization catalysts such as zinc compounds.

Other catalysts as mentioned later as representative of component D) may also be present when reacting components A1) to A4) to receive the prepolymer A1). However, it is preferred not to use a catalyst when reacting component A1) to A4).

The reaction is typically effected at 25°C to 140°C, preferably 60°C to 100°C.

If excess isocyanate has been employed, the excess of low molecular weight, preferably aliphatic diisocyanate is then removed, preferably by thin-film distillation using a thin-film evaporator.

Before, during and after the reaction or the distillative removal of the excess diisocyanate, preference is given to adding acidic or alkylating stabilizers, such as benzoyl chloride, isophthaloyl chloride, methyl tosylate, chloropropionic acid, HCl, dibutyl phosphate or antioxidants such as di-tert-butylcresol or tocopherol.

The NCO content of the isocyanate-functional prepolymers A) is preferably 1.5% to 8% by weight, more preferably 2% to 7.5% by weight and most preferably 3% to 7% by weight.

Examples of low molecular weight, aliphatic diisocyanates of component A1) are hexamethylene diisocyanate (HDI), isophorone diisocyanate (IPDI), butylene diisocyanate (BDI), pentamethylene diisocyanate (PDI), bisisocyanatocyclohexylmethane (HMDI), 2,2,4-trimethylhexamethylene diisocyanate, bisisocyanatomethylcyclohexane, bisisocyanatomethyltricyclodecane, xylene diisocyanate, tetramethylxylylene diisocyanate, norbornane diisocyanate, cyclohexane diisocyanate or diisocyanatododecane, preference being given to hexamethylene diisocyanate (HDI), isophorone diisocyanate (IPDI), butylene diisocyanate (BDI), pentamethylene diisocyanate (PDI), and bis(isocyanatocyclohexyl)methane (HMDI). Particular preference is given to hexamethylene diisocyanate and pentamethylene diisocyanate.

If aromatic diisocyanates are used as component A1, these are preferably toluene 2,4-diisocyanate (2,4-TDI), toluene 2,6-diisocyanate (2,6-TDI) or 2,2'-methylene diphenyl diisocyanate (2,2'-MDI), 2,4'-methylene diphenyl diisocyanate (2,4'-MDI), 4,4'-methylene diphenyl diisocyanate (4,4'-MDI), or mixtures of at least two of these.

Polyalkylene oxides of component A2) may be any polyalkylene oxides that the person skilled in the art would use for the purpose. Examples of these are selected from the group consisting of polyethylene oxide, polypropylene oxide, polytetrahydrofuran or a mixture of at least two of these. Polyalkylene oxides of component A2) are preferably copolymers of ethylene oxide and propylene oxide having an oxyethylene unit content, based on the total amount of the oxyalkylene groups present, of 50 to 100 mol%, preferably 60 to 90 mol%, started from polyols or amines. Preferred starters of this kind are selected from the group consisting of ethylene glycol, propane-1,3-diol, propane-1,2 diol (propylene glycol), dipropylene glycol, butane-1,4-diol, glycerol, trimethylolpropane (TMP), sorbitol, pentaerythritol, triethanolamine, ammonia and ethylenediamine, or a mixture of at least two of these.

The polyalkylene oxides of component A2) typically have number-average molecular weights of 250 to 10 000 g/mol, preferably of 300 to 2800 g/mol, more preferably 350 to 1500 g/mol.

In addition, the polyalkylene oxides of component A2) have OH functionalities of 2 to 6, preferably of 2 to 5, more preferably of 2 to 4.

Preferably, component A3) is selected from the group consisting of 1,2-ethandiol, 2-(2-hydroxyethoxy)ethan-1-ol, 1,2 propanediol, 1,3 propanediol, 1,2 butanediol, 1,3 butanediol, 1,4 butanediol, 1,2 pentanediol, 1,3 pentanediol, 1,4 pentanediol, 1,5 pentanediol, 1,2 hexanediol, 1,3 hexanediol, 1,4 hexanediol, 1,5 hexanediol, 1,6 hexanediol, 1,2 heptanediol, 1,3 heptanediol, 1,4 heptanediol, 1,5 heptanediol, 1,6 heptanediol, 1,7 heptanediol, 1,2 octanediol, 1,3 octanediol, 1,4 octanediol, 1,5 octanediol, 1,6 octanediol, 1,7 octanediol, 1,8 octanediol, 1,2 nonanediol, 1,3 nonanediol, 1,4 nonanediol, 1,5 nonanediol, 1,6 nonanediol, 1,7 nonanediol, 1,8 nonanediol, 1,9 nonanediol, 1,2 decanediol, 1,3 decanediol, 1,4 decanediol, 1,5 decanediol, 1,6 decanediol, 1,7 decanediol, 1,8 decanediol, 1,9 decanediol, 1,10 decanediol, 1,2 undecanediol, 1,3 undecanediol, 1,4 undecanediol, 1,5 undecanediol, 1,6 undecanediol, 1,7 undecanediol, 1,8 undecanediol, 1,9 undecanediol, 1,10 undecanediol, 1,11 undecanediol, 1,2 dodecanediol, 1,3 dodecanediol, 1,4 dodecanediol, 1,5 dodecanediol, 1,6 dodecanediol, 1,7 dodecanediol, 1,8 dodecanediol, 1,9 dodecanediol, 1,10 dodecanediol, 1,11 dodecanediol, 1,12 dodecanediol or mixtures of at least two thereof.

Preferably, component A3) is linear, most preferably selected from the group consisting of 1,2-ethandiol, 2-(2-hydroxyethoxy)ethan-1-ol, 1,3 propanediol, 1,4 butanediol, 1,6 hexanediol, 1,8 octanediol, 1,10 decanediol, 1,12 dodecanediol or mixtures of at least two thereof.

For component A4), in principle, preference is given to using any of the mono- and polyhydric alcohols or mono- and polyfunctional amines that are known per se to the person skilled in the art, and mixtures thereof. These are mono- or polyhydric alcohols or polyols that are different to those of the component A2) or A3), such as ethanol, propanol, butanol, decanol, tridecanol, hexadecanol, ethylene glycol, neopentyl glycol, trimethylolpropane, glycerol, pentaerythritol, monofunctional polyether alcohols and polyester alcohols, polyetherdiols and polyesterdiols or mixtures of at least two of these. Examples of mono- or polyfunctional amines include butylamine, ethylenediamine or amine-terminated polyalkylene glycols (e.g. Jeffamine®).

In the preparation of the prepolymer A), it is possible to use catalysts as described for component D) for example.

The water for use as component B) can be used as such, as water of crystallization in a salt, as a solution in a dipolar-aprotic solvent or else as an emulsion. Preference is given to using the water as such or in a dipolar-aprotic solvent. Very particular preference is given to using the water as such.

Any compounds of component C) that are optionally to be used are polyisocyanates obtainable by reaction of low molecular weight diisocyanates having a molar mass of 140 to 278 g/mol, such as biurets, oxadiazinetrione, allophanates, isocyanurates, iminooxadiazinediones or uretdiones of the aforementioned low molecular weight diisocyanates. Preference is given to using aliphatic diisocyanates for component C). Preferably, the 4-membered or 6-membered ring oligomers of low molecular weight diisocyanates have a functionality within a range from 2 to 6, or preferably within a range from 2.1 to 5.5, or preferably within a range from 2.5 to 5.

Examples of low molecular weight, preferably aliphatic diisocyanates of component C) are hexamethylene diisocyanate (HDI), isophorone diisocyanate (IPDI), butylene diisocyanate (BDI), pentamethylene diisocyanate (PDI), bisisocyanatocyclohexylmethane (HMDI), 2,2,4-trimethylhexamethylene diisocyanate, bisisocyanatomethylcyclohexane, bisisocyanatomethyltricyclodecane, xylene diisocyanate, tetramethylxylylene diisocyanate, norbornane diisocyanate, cyclohexane diisocyanate or diisocyanatododecane, preference being given to hexamethylene diisocyanate (HDI), isophorone diisocyanate (IPDI), butylene diisocyanate (BDI), pentamethylene diisocyanate (PDI), and bis(isocyanatocyclohexyl)methane (HMDI). Particular preference is given to PDI, HDI, IPDI, very particular preference to hexamethylene diisocyanate and pentamethylene diisocyanate.

If aromatic polyisocyanates are used as component C), these are preferably toluene 2,4-diisocyanate (2,4-TDI), toluene 2,6-diisocyanate (2,6-TDI) or 2,2'-methylene diphenyl diisocyanate (2,2'-MDI), 2,4'-methylene diphenyl diisocyanate (2,4'-MDI), 4,4'-methylene diphenyl diisocyanate (4,4'-MDI), or mixtures of at least two of these.

The content of isocyanate groups elevated by the use of component C) ensures better foaming since more $CO_2$ is formed in the isocyanate-water reaction.

To accelerate the urethane formation, catalysts can be used in component D). These are typically the compounds known to the person skilled in the art from polyurethane technology. Preference is given here to compounds from the group consisting of catalytically active metal salts, amines, amidines and guanidines. Examples include dibutyltin dilaurate (DBTL), tin acetate, 1,8-diazabicyclo[5.4.0]undecene-7 (DBU), 1,5-diazabicyclo[4.3.0]nonene-5 (DBN), 1,4-diazabicyclo[3.3.0]octene-4 (DBO), N-ethylmorpholine (NEM), triethylenediamine (DABCO), pentamethylguanidine (PMG), tetramethylguanidine (TMG), cyclotetramethylguanidine (TMGC), n-decyltetramethylguanidine (TMGD), n-dodecyltetramethylguanidine (TMGDO), dimethylaminoethyltetramethylguanidine (TMGN), 1,1,4,4,5,5-hexamethylisobiguanidine (HMIB), phenyltetramethylguanidine (TMGP) and hexamethyleneoctamethylbiguanidine (HOBG), dimethylaminopropylamine (DMAPA).

Preferably, the component C) has a residual diisocyanate content of below 1% by weight, preferably below 0.8% by weight, more preferably below 0.75% by weight, especially preferably below 0.5% by weight, based on the total weight of the component C).

Preference is given to the use of amines, amidines, guanidines or mixtures thereof as catalysts of component D). In addition, preference is also given to the use of 1,8-diazabicyclo[5.4.0]undecene-7 (DBU), 1,5-diazabicyclo[4.3.0]nonene-5 (DBN), triethylenediamine (DABCO).

It is preferred not to use catalysts at all.

As component E) it is optionally possible to use salts of weak acids, the corresponding free acids of which have a pKA in water at 25°C of ≥ 3.0 and ≤ 14.0. Examples of suitable salts of weak acids are potassium hydroxide, sodium hydroxide, potassium carbonate, sodium carbonate and sodium hydrogencarbonate, sodium acetate, potassium acetate, sodium citrate, potassium citrate, sodium benzoate, potassium benzoate, also including any desired mixtures of these salts. It is preferable when the salts of weak acids are selected from the group of sodium hydroxide, sodium hydrogencarbonate and sodium carbonate. In this case, the result is a particularly short reaction time.

In the case of forming a foam, to improve foam formation, foam stability or the properties of the resulting polyurethane foam, compounds of component F) may be used, where such additives may in principle be any of the anionic, cationic, amphoteric and nonionic surfactants that are known per se, and mixtures of these. Preference is given to using alkyl polyglycosides, EO/PO block copolymers, alkyl or aryl alkoxylates, siloxane alkoxylates, esters of sulfosuccinic acid and/or alkali metal or alkaline earth metal alkanoates or mixtures of at least two of these. Particular preference is given to using EO/PO block copolymers. Preference is given to using solly the EO/PO block copolymers as component F).

In addition, to improve the foam or hydrogel properties of the resulting polyurethane foam, compounds of component G) may be used. These are in principle all the mono- and polyhydric alcohols that are known per se to the person skilled in the art, and mixtures of these. These are mono- or polyhydric alcohols or polyols, such as ethanol, propanol, butanol, decanol, tridecanol, hexadecanol, ethylene glycol, neopentyl glycol, butanediol, hexanediol, decanediol, trimethylolpropane, glycerol, pentaerythritol, monofunctional polyether alcohols and polyester alcohols, polyetherdiols and polyesterdiols or mixtures of at least two of these.

In the preparation of the hydrophilic polyisocyanates H), the ratio of the monohydroxyfunctional polyalkylene oxides H2) to the low molecular weight diisocyanates H1) is typically adjusted such that, for every 1 mol of OH groups of the monohydroxyfunctional polyalkylene oxides, there are 1.25 to 20 mol, preferably 2 to 15 mol and more preferably 5 to 13 mol of NCO groups of the low molecular weight diisocyanate H1). This is followed by allophanatization or biuretization and/or isocyanurate formation or uretdione formation. If the polyalkylene oxides H2) are bonded to the preferably aliphatic diisocyanates H1) via urethane groups, allophanatization preferably takes place thereafter. It is further preferable that isocyanate structural units are formed.

A preferred alternative preparation of the hydrophilic polyisocyanates H) is typically effected by reaction of 1 mol of OH groups of the monohydroxyfunctional polyalkylene oxide component H2) with 1.25 to 20 mol, preferably with 2 to 15 mol and more preferably 5 to 13 mol of NCO groups of a polyisocyanate H1) having an isocyanate functionality of 2 to 6, based on aliphatic or aromatic diisocyanates, preferably aliphatic diisocyanates. Examples of such polyisocyanates H1) are biuret structures, isocyanurates or uretdiones based on preferably aliphatic diisocyanates. The polyisocyanate H1) and the polyalkylene oxide H2) are preferably joined to one another via a urethane group or a urea group, preference being given particularly to linkage via urethane groups.

Preferably, the component H) has a residual diisocyanate content of below 1% by weight, preferably below 0.8% by weight, more preferably below 0.75% by weight, especially preferably below 0.5% by weight, based on the total weight of the component H).

[A2] In a preferred embodiment, component A) comprises component A3) within a range from 1.1 to 4.9% by weight, more preferably in a range from 1.2 to 4.7% by weight, even more preferably in a range from 1.5 to 4.5% by weight, based on the total amount of the isocyanate-reactive components, at least based on the total amount of the isocyanate-reactive components A2) to A4).

[A3] In a preferred embodiment the isocyanate-containing components, especially components A), C) and H), have a total isocyanate content within a range from 2% to 12% by weight, more preferably in a range from 2% to 10% by weight, even more preferably in a range from 2% to 8% by weight, based on the total amount of the isocyanate-containing components.

Preferably, the isocyanate-containing components, especially components A), C) and H) have a content of urethane groups of 1.0 to 3.5 mol/kg, based on the total amount of the isocyanate-containing components. Preferably, the isocyanate-containing components, especially components A), C) and H), have a total isocyanate content within a range from 3% to 7% or preferably within a range from 4% to 6.5% by weight, and preferably a content of urethane groups within a range from 1.5 to 3.0 mol/kg, or preferably within a range from 1.7 to 2.8 mol/kg, based in each case on the total amount of the isocyanate-containing components.

Preference is given to using aliphatic diisocyanates in the production of polyurethane foams or hydrogels of the invention. More particularly, preference is given to using exclusively aliphatic diisocyanates for components A1), C) and H1).

Preference is given to using linear diisocyanates in the production of polyurethane foams or hydrogels of the invention. More particularly, preference is given to using exclusively linear diisocyanates for components A1), C) and H1).

The reaction can be effected in the presence of urethanization catalysts such as tin compounds, zinc compounds, amines, guanidines or amidines, or in the presence of allophanatization catalysts such as zinc compounds.

The reaction is typically effected at 25°C to 140°C, preferably at 60°C to 100°C.

If excess low molecular weight diisocyanate has been employed, the excess of low molecular weight, preferably aliphatic diisocyanate is then removed, preferably by thin-film distillation.

Before, during and/or after the reaction or the distillative removal of the excess diisocyanate, it is possible to add acidic or alkylating stabilizers, such as benzoyl chloride, isophthaloyl chloride, methyl tosylate, chloropropionic acid, HCl or antioxidants such as di-*tert*-butylcresol or tocopherol.

The NCO content of the hydrophilic polyisocyanates H) is preferably 0.3% to 23% by weight, more preferably 2% to 21% by weight and most preferably 3% to 18% by weight.

Examples of low molecular weight, preferably aliphatic diisocyanates of component H1) are hexamethylene diisocyanate (HDI), isophorone diisocyanate (IPDI), butylene diisocyanate (BDI), pentamethylene diisocyanate (PDI), bisisocyanatocyclohexylmethane (HMDI), 2,2,4-trimethylhexamethylene diisocyanate, bisisocyanatomethylcyclohexane, bisisocyanatomethyltricyclodecane, xylene diisocyanate, tetramethylxylylene diisocyanate, norbornane diisocyanate, cyclohexane diisocyanate or diisocyanatododecane, preference being given to hexamethylene diisocyanate (HDI), isophorone diisocyanate (IPDI), butylene diisocyanate (BDI), pentamethylene diisocyanate (PDI), and bis(isocyanatocyclohexyl)methane (HMDI). Particular preference is given to PDI, HDI, IPDI, very particular preference to hexamethylene diisocyanate and pentamethylene diisocyanate.

If aromatic diisocyanates are used as component H1), these are preferably toluene 2,4-diisocyanate (2,4-TDI), toluene 2,6-diisocyanate (2,6-TDI) or 2,2'-methylene diphenyl diisocyanate (2,2'-MDI), 2,4'-methylene diphenyl diisocyanate (2,4'-MDI), 4,4'-methylene diphenyl diisocyanate (4,4'-MDI), or mixtures of at least two of these.

Examples of higher molecular weight polyisocyanates H2) are polyisocyanates having an average isocyanate functionality of 2 to 6 with isocyanurate, urethane, allophanate, biuret, iminooxadiazinetrione, oxadiazinetrione and/or uretdione groups, based on the preferably aliphatic and/or cycloaliphatic diisocyanates mentioned in the paragraph above.

Components H2) used are preferably higher molecular weight compounds with biuret, iminooxadiazinedione, isocyanurate and/or uretdione groups, based on hexamethylene diisocyanate, isophorone diisocyanate and/or 4,4'-diisocyanatodicyclohexylmethane. Preference is further given to isocyanurates. Very particular preference is given to structures based on hexamethylene diisocyanate.

The monohydroxyfunctional polyalkylene oxides H2) have an OH number of 15 to 250, preferably of 28 to 112, and an oxyethylene unit content of 50 to 100 mol%, preferably of 60 to 100 mol%, based on the total amount of the oxyalkylene groups present.

Monohydroxyfunctional polyalkylene oxides in the context of the invention are understood to mean compounds that have only one isocyanate-reactive group, i.e. one group that can react with an NCO group.

The preparation of polyalkylene oxides H2) by alkoxylation of suitable starter molecules is known from the literature (e.g. Ullmanns Encyclopädie der technischen Chemie [Ullmann's Encyclopedia of Industrial Chemistry], 4th edition, volume 19, Verlag Chemie, Weinheim p. 31-38). Suitable starter molecules are especially saturated monoalcohols such as methanol, ethanol, n-propanol, isopropanol, n-butanol, isobutanol, sec-butanol, diethylene glycol monobutyl ether, and aromatic alcohols such as phenol or monoamines such as diethylamine. Preferred starter molecules are saturated monoalcohols of the aforementioned type. Particular preference is given to using diethylene glycol monobutyl ether or n-butanol as starter molecules.

The monohydroxyfunctional polyalkylene oxides H2) typically have number-average molecular weights of 220 to 3700 g/mol, preferably of 250 to 2800 g/mol, or preferably of 300 to 2000 g/mol.

The monohydroxyfunctional polyalkylene oxides H2) preferably have an OH group as isocyanate-reactive group.

Typically, components A) to H) are used in the following amounts:

100 parts by weight of isocyanate-functional prepolymers A)

0.1 to 200 parts by weight of water or a nonaqueous isocyanate-reactive component B)

0 to 30 parts by weight of heterocyclic oligomers C)

0 to 1 part by weight of catalysts D)

0 to 5 parts by weight of salts of weak acids, the corresponding free acids of which have a pKA in water at 25°C of ≥ 3.0 and ≤ 14.0 E)

0 to 10 parts by weight of surfactants F)

0 to 20 parts by weight of alcohols G)

0.5 to 50 parts by weight of hydrophilic polyisocyanate component H)

Preferably, components A) to H) are used in the following amounts:

100 parts by weight of isocyanate-functional prepolymers A)

0.1 to 100 parts by weight of water or a nonaqueous isocyanate-reactive component B)

1 to 20 parts by weight of heterocyclic oligomers C)

0 to 1 part by weight of catalysts D)

0 to 5 parts by weight of salts of weak acids, the corresponding free acids of which have a pKA in water at 25°C of $\geq$ 3.0 and $\leq$ 14.0 E)

0 to 5 parts by weight of surfactants F)

0 to 10 parts by weight of alcohols G)

1 to 25 parts by weight of hydrophilic polyisocyanates H)

More preferably, components A) to H) are used in the following amounts:

100 parts by weight of isocyanate-functional prepolymers A)

1 to 60 parts by weight of water or a nonaqueous isocyanate-reactive component B)

2 to 15 parts by weight of heterocyclic oligomers C)

0 to 0.5 part by weight of catalysts D)

0 part by weight of salts of weak acids, the corresponding free acids of which have a pKA in water at 25°C of $\geq$ 3.0 and $\leq$ 14.0 E)

0 to 3 parts by weight of surfactants F)

0 part by weight of alcohols G)

2 to 15 parts by weight of hydrophilic polyisocyanates H)

[A4] In a preferred embodiment of the process according to the invention, prepolymer A) has a proportion by weight of low molecular weight diisocyanates having a molar mass of 140 to 278 g/mol of below 1.0% by weight, preferably of below 0.5% by weight, preferably of below 0.3% by weight, or preferably of below 0.1% by weight, based on the Prepolymer A). The proportion by weight of low molecular weight diisocyanates is preferably adjusted via distillation.

[A5] In a preferred embodiment of the process according to the invention, aliphatic isocyanates are used as component A1). Examples of aliphatic isocyanates have already been mentioned above.

[A6] In a preferred embodiment of the process according to the invention, linear diisocyanates in the production of polyurethane foams or hydrogels are used. More particularly, preference is given to using exclusively linear diisocyanates for components A1), C) and H1).

[A7] In a preferred embodiment of the process according to the invention, unbranched diols in the production of polyurethane foams or hydrogels are used as component A3). Preferred examples of component A3) have been mentioned above.

[A8] In a preferred embodiment of the process according to the invention, the isocyanate-functional prepolymer A has a viscosity of $\leq$ 50 000 mPas, preferably of $\leq$ 25 000, or preferably of $\leq$ 10 000, or preferably within a range from 100 to 20 000 mPas.

[A9] In a preferred embodiment of the process according to the invention, at least the following steps are conducted:

I) preparing the prepolymer A) from components A1), A2), A3) and optionally A4), optionally D),

II) optionally mixing components A), C) and H) and other isocyanate-containing components to obtain a prepolymer mixture,

III) optionally adding A4) and optionally D),

IV) optionally mixing component B) with all other components, especially D), E), F) and G), apart from the prepolymer mixture,

V) mixing the prepolymer mixture obtained in I) to III) with the mixture from IV).

Preferably, the temperature in at least one of steps I) to IV) is chosen within a range from 2 to 70°C, or preferably within a range from 10 to 50°C, or preferably from 20 to 40°C.

Preferably, the mixture, after step IV), is applied to a substrate and allowed to cure. The curing is preferably conducted at a temperature within a range from 20 to 50°C. With the aid of convection ovens or infrared dryers, the curing and simultaneous drying can also be conducted in higher temperature ranges, for example between 50 and 200°C.

The polyurethane foams or hydrogels according to the invention are preferably produced by mixing components A), produced from components A1), A2), A3) and optionally A4) and optionally D), optionally with C) and/or H), in any sequence, and then with a mixture of B) and optionally D), E), F), G), foaming the mixture and curing, preferably by chemical crosslinking. Preferably, components A), C) and H) are pre-mixed with one another. Any salts E) to be used and any surfactants F) are preferably added to the reaction mixture in the form of their aqueous solutions.

The foaming can in principle be effected by means of the carbon dioxide formed in the reaction of the isocyanate groups with water, but the use of other blowing agents is likewise possible. For instance, it is also possible in principle to use blowing agents from the class of the hydrocarbons such as $C_3$-$C_6$-alkanes, e.g. butanes, *n*-pentane, *iso*-pentane, *cyclo*-pentane, hexanes or the like or halogenated hydrocarbons such as dichloromethane, dichloromonofluoromethane, chlorodifluoroethanes, 1,1-dichloro-2,2,2-trifluoroethane, 2,2-dichloro-2-fluoroethane, especially chlorine-free hydrofluorocarbons such as difluoromethane, trifluoromethane, difluoroethane, 1,1,1,2-tetrafluoroethane, tetrafluoroethane (R 134 or R 134a), 1,1,1,3,3-pentafluoropropane (R 245 fa), 1,1,1,3,3,3-hexafluoropropane (R 256), 1,1,1,3,3-pentafluorobutane (R 365 mfc), heptafluoropropane or else sulfur hexafluoride. Mixtures of these blowing agents are also usable.

The subsequent curing is typically effected at room temperature.

[A10] In a preferred embodiment of the process according to the invention, the oxyethylene unit content of A2 is $\geq$ 50% by weight, or preferably $\geq$ 55% by weight, or preferably $\geq$ 60% by weight, based on the total amount of the oxyalkylene groups present.

Preferably, there is a molar ratio of NCO groups to OH groups in the reaction of components A1) to A4) to give the isocyanate-functional prepolymer A) of < 5, or preferably of < 4, or preferably of < 3, or preferably within a range from 1 to 5, or preferably within a range from 1.5 to 4.5.

[A11] In a preferred embodiment of the process according to the invention, the polyalkylene oxide A2) has an OH number within a range from 25 to 450 mg KOH/g, preferably within a range from 50 to 400, or preferably within a range from 75 to 250.

Moreover, the present invention further provides the polyurethane foams or hydrogels produced by the process according to the invention, and for the use of the hydrophilic, preferably aliphatic polyurethane foams or hydrogels as a constituent of a wound dressing, a cosmetic article or an incontinence product.

As already described above, the polyurethane foams or hydrogels according to the invention are preferably produced by mixing components A), produced from components A1), A2), A3) and optionally A4), optionally D), optionally with C) and/or H), in any sequence, and then with a mixture of B) and optionally D), E), F), G), foaming the mixture and curing, preferably by chemical crosslinking. Preferably, components A), C) and H) are pre-mixed with one another. Any salts E) to be used and any surfactants F) are preferably added to the reaction mixture in the form of their aqueous solutions. Preferably, the polyurethane foams or hydrogels according to the invention are hydrophilic and have aliphatic units.

In the case of forming foams, the polyurethane foams according to the invention or the polyurethane foams produced in accordance with the invention preferably have a porous, at least partly open-cell structure with cells in communication with one another. The density of the polyurethane foams is preferably 50 to 300 g/l.

The polyurethane foams have good mechanical strength and high elasticity. Typically, tensile $strength_{dry}$ values are greater than 40 kPa, elongation at break values greater than 30% with a density in the range from 50 to 300 g/l (determination in each case by the standards as described later under Methods).

After the production, the polyurethane foams can be processed by methods known per se to give essentially two-dimensional or thin three-dimensional materials which can then be used, for example, as a constituent of a wound dressing, of a cosmetic article or of an incontinence product. In general, for this purpose, slabstock foams are cut to the desired thickness by standard methods, to obtain two-dimensional materials having a thickness of typically from 10 $\mu$m to 5 cm, preferably from 0.1 mm to 1 cm, more preferably from 0.1 mm to 6 mm, most preferably from 0.2 mm to 6 mm

With the aid of suitable casting techniques, the two-dimensional materials described can alternatively be obtained directly by application and optionally foaming of the composition according to the invention to a substrate, for example an optionally pretreated paper, a film, a nonwoven or a textile.

In a preferred variant, for this purpose, a mixture of the starting materials as described in the PCT application numbered WO 2011/006608 is applied to a substrate by means of a coating bar, and then the optional foaming follows after the coating. The gap height of the coating bar is generally in the range from 0.2 to 20 mm, preferably from 0.5 to 5 mm and most preferably from 0.8 to 2 mm. The film width of the coating bar to be used can be matched to the particular end use. Examples are film widths between 10 and 5000 mm, preferably between 20 and 2000 mm

Preference is given to a casting method in which, while the polyurethane foam or hydrogels is being cast to a layer or a substrate, a further layer is applied to the top side of the polyurethane foam or hydrogel, preferably before the polyurethane foam or hydrogel has dried. Such a process is described in the patent application with application number EP 17156493.3 and can likewise be employed here.

The polyurethane foams generally contain only a small water-extractable proportion of not more than 2% by weight, preferably of not more than 1% by weight, meaning that they contain only very small amounts of chemically unbound constituents.

Moreover, the polyurethane foams or hydrogels may be bonded, laminated or coated with further materials, for example based on hydrogels, (semi-)permeable films, foam films, coatings, hydrocolloids or other foams.

The polyurethane foams or hydrogels according to the invention are particularly suitable for production of wound dressings. The polyurethane foams or hydrogels here may be in direct or indirect contact with the wound. However, preference is given to using the polyurethane foams or hydrogels in direct contact with the wound in order to assure, for example, optimal absorption of wound fluid.

The polyurethane foams or hydrogels that are used as wound dressing must additionally be sterilized in a further process step. For sterilization, the processes known per se to the person skilled in the art are used, in which sterilization is effected by thermal treatment, chemical substances such as oxyethylene, or irradiation, for example by gamma irradiation. The irradiation can optionally be effected under protective gas atmosphere. The polyurethane foams or hydrogels according to the invention made from preferably aliphatic diisocyanates, especially component A1) and optionally H1), have the great advantage that they are not discoloured on irradiation, especially on irradiation with gamma rays.

Likewise possible is addition, incorporation or coating of or with antimicrobial, pharmaceutical or biological active ingredients or other additives that have a positive effect, for example in relation to wound healing and the avoidance of microbial contamination.

[A12] A further aspect of the invention is a polyurethane foam or polyurethane hydrogel which as obtained according to the process according to the invention as described above.

[A13] The invention further provides a polyurethane prepolymer mixture comprising the following components:

A) a polyurethane prepolymer obtainable from

A1) low molecular weight diisocyanates of molar mass from 140 to 278 g/mol, with

A2) polyalkylene oxides having an OH functionality of two or more, preferably within a range from 2 to 6, or preferably within a range from 2.1 to 5,

A3) more than 1% by weight of $C_2$ to $C_{12}$ diols based on the total amount of the isocyanate-reactive components A2) to A4) wherein component A3) differs from component A2), A4) optionally further isocyanate-reactive components differing from A2) and A3);

C) optionally polyisocyanates obtainable by reaction of at least two low molecular, preferably aliphatic diisocyanates, wherein the diisocyanates having a molar mass of 140 to 278 g/mol; optionally polyisocyanates obtainable by reaction of at least two low molecular, preferably aliphatic diisocyanates, wherein the diisocyanates having a molar mass of 140 to 278 g/mol, preferably heterocyclic 4-membered or 6-membered ring oligomers of low molecular weight diisocyanates having a molar mass of 140 to 278 g/mol, preferably having an isocyanate functionality of 2 to 6, or an isocyanate functionality of 2.1 to 5;

D) optionally catalysts;

H) optionally hydrophilic polyisocyanates obtainable by reaction of

H1) low molecular weight diisocyanates of molar mass from 140 to 278 g/mol and/or polyisocyanates preparable therefrom and having an average isocyanate functionality of 2 to 6, or an isocyanate functionality of 2.1 to 5;

H2) monohydroxyfunctional polyalkylene oxides of OH number from 10 to 250, or preferably of 20 to 200, and of oxyethylene unit content from 50 to 100 mol%, based on the total amount of the oxyalkylene groups present.

wherein the isocyanate containing components, specifically components A), C) and H), do not exceed a residual diisocyanate content of 8% by weight, more preferably below 5% by weight, even more preferably below 3% by weight, especially preferably below 2%, even more preferably below 0.5% by weight, most preferably below 0.1% by weight based on the total amount of the polyurethane foam or hydrgel.

Preferably, the prepolymers A) used have a residual diisocyanate content of below 0.5% by weight, based on the total weight of the prepolymer.

In the preparation of the isocyanate-functional prepolymers A), the amounts of polyalkylene oxides A2), the $C_2$ to $C_{12}$ diols A3), A4) and the low molecular weight, preferably aliphatic diisocyanates A1) are typically adjusted such that, for every 1 mol of OH groups of the combined components A2), A3) and A4), there are 1.1 to 20 mol, preferably 1.5 to 10 mol and more preferably 2 to 5 mol of NCO groups of the low molecular weight, preferably aliphatic diisocyanate A1).

In the preparation of the isocyanate-functional prepolymers A), the amounts of polyalkylene oxides A2), the $C_2$ to $C_{12}$ diols A3), the isocyanate reactive components A4) and the low molecular weight, preferably aliphatic diisocyanates A1) are typically adjusted such that, for every 1 mol of isocyanate reactive groups of the combined components A2), A3), and A4), there are 1.1 to 20 mol, preferably 1.5 to 10 mol and more preferably 2 to 5 mol of NCO groups of the low molecular weight, preferably aliphatic diisocyanate A1).

All selections, amounts, properties and ratios mentioned in connection with the components A), A1), A2), A3), A4), C), D), E), F), G) and H) for the process according to the invention for producing polyurethane foams or hydrogels above are also applicable for the polyurethane prepolymer mixture according to the invention. To achieve a foam or hydrogel starting from the polyurethane prepolymer mixture component B) as mentioned in connection with process according to the invention is mixed with the polyurethane prepolymer mixture.

Preferably, the polyurethane prepolymer mixture, especially components A), C) and H), has an isocyanate content within a range from 2% to 8% by weight, or preferably within a range from 3% to 7%, or preferably within a range from 4% to 6.5% by weight, and a content of urethane groups within a range from 1.0 to 3.5 mol/kg, or preferably within a range from 1.5 to 3.0 mol/kg, or preferably within a range from 1.7 to 2.8 mol/kg, based in each case on the total amount of the polyurethane prepolymer mixture.

The polyurethane prepolymer mixture according to the invention is preferably converted to the polyurethane foam or hydrogel as in the above-described process for producing a polyurethane foam or hydrogel. All the components mentioned for the polyurethane prepolymer mixture have the same properties as already described for these components in connection with the process according to the invention.

The invention further relates to the use of the polyurethane prepolymer mixture according to the invention or of the polyurethane foam or hydrogel according to the invention for production of a wound dressing, a cosmetic article or an incontinence product.

### *Raw materials*

### Catalyst for alkylene oxide addition (DMC-catalyst)

Double metal cyanide catalyst, containing zinc hexacyanocobaltate, tert-butanol and polypropylene glycol having a number-average molar mass of 1000 Da; described in WO-A 01/80994, example 6.

### IRGANOX® 1076

Octadecyl 3-(3,5-di-tert-butyl-4-hydroxyphenyl)propionate (BASF SE)

### Desmodur® 24 MI

Desmodur® 24 MI is a monomeric diphenylmethane-2,4'-diisocyanate (MDI). The content of diphenylmethane-2,4'-diisocyanate is > 97.5% by weight.

**Desmodur® 44 M**

Desmodur® 44 M Liquid is a monomeric diphenylmethane-4,4'-diisocyanate (MDI). The content of diphenylmethane-4,4'-diisocyanate is > 98.2% by weight.

**Desmophen® L 300**

Polyethylene glycol, prepared by ethoxylation of propylene glycol, OH-number: 190 mg KOH / g, commercial product of Covestro Deutschland AG

**Poly(ethyleneglycol-ran-propylenglycol)**

Poly(ethyleneglycol-ran-propylenglycol) is a difunctional polyether (MERCK). The molar mass and fraction of ethylene oxide units were determined via $^1$H-NMR spectroscopy in deutorated chloroform solvent to be 1870 g/mol and 80.4%, respectively.

**Dipropylene glycol**

Product of INEOS; minimum purity: 99.7 %; water content 1000 ppm (max.)

**Low molecular weight polyols**

1,2-Ethanediol (KRAFT, 99%), 1,4-Butanediol (MERCK, > 99%), 1,6-Hexanediol (MERCK, 97%) and 1,12-Dodecanediol (MERCK, 99%), and Trimethylol propane (LANXESS) were used as received.

*Methods*

Percentages denote weight percentages unless stated otherwise.

**Viscosity**

Viscosities of prepolymers were determined according to DIN 53019. Unless stated otherwise the temperature was 23 °C.

**OH-numbers**

The OH numbers were determined according to the method of DIN 53240.

**Molecular weight distribution**

The molecular weight distribution was determined by means of size exclusion chromatography (SEC). The apparatus Agilent 1100 Series from Agilent was used. The polydispersity PD for the molecular weight distribution Mw/Mn, wherein Mw represents the weight-average molecular weight and Mn represents the number-average molecular weight, is stated. Further information on this analysis:

- Column combination: 1 pre-column PSS, 5 $\mu$l, 8 x 50 mm; 2 PSS SVD, 5 $\mu$l, 100 Å, 8 x 300 mm; 2 PSS SVD, 5 $\mu$l, 1,000 Å, 8 x 300 mm, PSS is the manufacturer of the columns (Polymer Standard Solutions, Mainz)
- Evaluation software: WIN GPC from PSS
- Solvent: THF (Merck LiChrosolv)
- Flow rate: 1 ml/min
- Detector type: RI detector (refractive index), Shodex RI 74
- Calibration standards used: calibration standard from PSS based on polystyrene.

**NCO content**

NCO contents were determined according to DIN-EN ISO 11909:2007.

**Residual diisocyanate content**

Residual diisocyanate contents were determined according to DIN EN ISO 10283:2007.

**Foam/Hydrogel height**

The height of foam samples was measured by a compressed air caliper connected to a display (Heidehain MT25P).

**Measurement of foam/hydrogel density (D)**

Foam samples of the dimensions 5 x 5 cm were punched out of the foam sheet. The sample height was determined as described previously at five locations and averaged. Subsequently, the samples were weighed using a Mettler Toledo XS603S balance and the density (D) was calculated.

**Measurement of swelling (edge length expansion factor (S))**

Immediately after measuring the liquid absorption according to DIN EN 13726-1:2002 the edge lengths of the swollen samples were determined. From the resulting 4 values an average edge length was calculated. The edge length expansion factor S was calculated as follows

$$S = (\text{average edge length after swelling in cm})/5\text{cm}$$

**Elongation at break and tensile strength at break**

Elongation at break ($\varepsilon_{b,wet}$) and wet-tensile-strength-at-break ($\sigma_{b,wet}$) in the wet (swollen) state were determined according to DIN EN ISO 527-2 except for the following deviations. The samples were immersed in deionized water for 30 min prior to punching out the tensile specimens. The wet specimen height needed for the calculation of the wet-tensile-strength-at-break ($\sigma_{b,wet}$) could not be measured reliably due to the caliper sinking into the extremely soft specimen. Therefore, the wet foam height was estimated from the dry foam height and the edge length expension according to the formula

$$h(wet\ sample) = h(dry\ sample) \cdot edge\ length\ expansion\ factor$$

*Examples*

**Polyether 1**

A 10 L laboratory autoclave was charged with 4126 g of L300 and 0.215 g of DMC catalyst. The autoclave was closed and air was exchanged against nitrogen by 3 times evacuating the autoclave down to 10 mbar and re-filling it with nitrogen up to 4.5 bar. Then the pressure was lowered again with stirring (propeller agitator, 130 rpm) and the autoclave was heated to 130 °C. The contents of the autoclave were stripped for 30 min. at 130 °C while continuously stirring in vacuum under an absolute pressure from 100 to 120 mbar, while passing in 50 ml of nitrogen per minute via a distributor ring lying under the level of the liquid. Then, the pressure was adjusted to 2.1 bar, the agitator speed was increased to 450 rpm and a blend of 49.0 g of propylene oxide (PO) and 146.5 g of ethylene oxide (EO) was fed into the autoclave within 14 min. The pressure rose to 4.8 bar. The activation of the DMC catalyst was indicated by an accelerated pressure decrease and the feed of a blend of 669.5 g of PO and 2001.4 g of EO could be resumed when the pressure had fallen to 2.3 bar. The epoxides were metered into the autoclave within 2.9 h. After a post reaction time of 20 min. the autoclave was evacuated under stirring at 130 °C to 12 mbar for 30 min. After cooling to 80 °C 2.407 g of IRGANOX® 1076 were added.

The product's OH-number was 112 mg KOH / g and the viscosity was 206 mPas at 25 °C. Its appearance was clear and colorless. PD = 1.04.

**Precursor for Polyether 2**

A 2 l laboratory autoclave was charged with 446.7 g of dipropylene glycol and 0.344 g of a 44.89 wt.-% solution of KOH in water. The autoclave was closed and air was exchanged against nitrogen at ambient temperature by 5 times evacuating the autoclave down to 50 mbar and re-filling it with nitrogen up to 2.0 bar. The agitator speed (propeller) was adjusted to 800 rpm and the autoclave was heated to 150 °C. The pressure was adjusted to 2.7 bar. 1053.1 g of EO were metered into the autoclave under agitation within 9.2 h. After a post reaction time of 4 h the autoclave was evacuated under stirring at 150 °C to 20 mbar for 30 min. After cooling to 80 °C, 2.151 g of sulfuric acid (11.8 wt.-% in water) were added, followed by the addition of 0.766 g of IRGANOX® 1076. Eventually, the product was evacuated to 20 mbar at 110 °C for 3 h under stirring.

The precursor's OH-number was 251 mg KOH / g and the viscosity was 93 mPas at 25 °C. Its appearance was clear and slightly yellowish. PD = 1.10.

**Polyether 2**

A 2 l laboratory autoclave was charged with 670.3 g of the precursor and 0.141 g of DMC catalyst. The autoclave was closed and air was exchanged against nitrogen by 3 times evacuating the autoclave down to 30 mbar and re-filling it with nitrogen up to 2.3 bar. Then the pressure was lowered again with stirring (propeller agitator, 150 rpm) and the autoclave was heated to 130 °C. The contents of the autoclave were stripped for 65 min. at 130 °C while continuously stirring in vacuum under an absolute pressure from 140 to 160 mbar, while passing in 50 ml of nitrogen per minute via a distributor ring lying under the level of the liquid. The agitator speed was increased to 800 rpm and a blend of 207.5 g of PO and 622.4 g of EO was fed into the autoclave within 3.5 h. The maximum pressure reached during the epoxide feed was 1.4 bar. After a post reaction time of 15 min. the autoclave was evacuated under stirring at 130 °C to 35 mbar for 80 min. After cooling to 80 °C 0.763 g of IRGANOX® 1076 were added.

The product's OH-number was 111 mg KOH / g and the viscosity was 188 mPas at 25 °C. Its appearance was clear and colorless. PD = 1.07

**Prepolymer 1 (inventive)**

316 g of HDI and 1.1 g of dibutyl phosphate were heated to 80 °C. Over the course of 1 h a mixture of 12.5 g 1,4-butane diol and 487.5 g of polyether 1 were added dropwise while stirring. The reaction mixture stirred at 80 °C for another 2.5 h until an NCO content of 12.8% was reached. Excess HDI was removed via distillation employing a thin film evaporator at 130 °C and about 0.7 mbar. A turbid prepolymer with an NCO content of 5.1%, a viscosity of 3330 mPas and a residual diisocyanate content of 0.01% was obtained.

**Prepolymer 2 (inventive)**

307 g of HDI and 0.8 g of dibutyl phosphate were heated to 80 °C. Over the course of 1 h a mixture of 17.5 g 1,4-butane diol and 482.5 g of polyether 1 were added dropwise while stirring. The reaction mixture stirred at 80 °C for another 2.5 h until an NCO content of 12.0% was reached. Excess HDI was removed via distillation employing a thin film evaporator at 130 °C and about 0.7 mbar. A turbid prepolymer with an NCO content of 5.4%, a viscosity of 4590 mPas and a residual diisocyanate content of 0.02% was obtained.

**Prepolymer 3 (inventive)**

287 g of HDI and 0.7 g of dibutyl phosphate were heated to 80 °C. Over the course of 1 h a mixture of 10.33 g 1,2-ethane diol and 403 g of polyether 1 were added dropwise while stirring. The reaction mixture stirred at 80 °C for another 2.5 h until an NCO content of 13.7% was reached. Excess HDI was removed via distillation employing a thin film evaporator at 130 °C and about 0.7 mbar. A turbid prepolymer with an NCO content of 5.4%, a viscosity of 2830 mPas and a residual diisocyanate content of 0.03% was obtained.

**Prepolymer 4 (inventive)**

289.5 g of HDI and 0.7 g of dibutyl phosphate were heated to 80 °C. Over the course of 1 h a mixture of 10.26 g 1,6-hexane diol and 400.2 g of polyether 1 were added dropwise while stirring. The reaction mixture stirred at 80 °C for another 2.5 h until an NCO content of 14.7% was reached. Excess HDI was removed via distillation employing a thin film evaporator at 130 °C and about 0.7 mbar. A turbid prepolymer with an NCO content of 5.4%, a viscosity of 2100 mPas and a residual diisocyanate content of 0.05% was obtained.

**Prepolymer 5 (inventive)**

318.0 g of HDI and 0.8 g of dibutyl phosphate were heated to 80 °C. Over the course of 1 h a mixture of 17.50 g 1,6-hexane diol and 482.5 g of polyether 1 were added dropwise while stirring. The reaction mixture stirred at 80 °C for another 2.5 h until an NCO content of 12.6% was reached. Excess HDI was removed via distillation employing a thin film evaporator at 130 °C and about 0.7 mbar. A turbid prepolymer with an NCO content of 5.2%, a viscosity of 2970 mPas and a residual diisocyanate content of 0.02% was obtained.

**Prepolymer 6 (inventive)**

269.5 g of HDI and 0.7 g of dibutyl phosphate were heated to 80 °C. Over the course of 1 h a mixture of 10.76 g 1,4-butane diol and 419.7 g of polyether 2 were added dropwise while stirring. The reaction mixture stirred at 80 °C for another 2.5 h until an NCO content of 12.8% was reached. Excess HDI was removed via distillation employing a thin film evaporator at 130 °C and about 0.7 mbar. A turbid prepolymer with an NCO content of 5.4%, a viscosity of 3280 mPas and a residual diisocyanate content of 0.03% was obtained.

**Prepolymer 7 (inventive)**

408.2 g of HDI and 1.0 g of dibutyl phosphate were heated to 80 °C. Over the course of 1 h a mixture of 14.80 g 1,2-ethane diol and 577.0g of Desmophen L 300 were added dropwise while stirring. The reaction mixture stirred at 80 °C for another 2.5 h until an NCO content of 10.2% was reached. Excess HDI was removed via distillation employing a thin film evaporator at 130 °C and about 0.8 mbar. A turbid prepolymer with an NCO content of 5.7%, a viscosity of 5650 mPas and a residual diisocyanate content of 0.04% was obtained.

**Prepolymer 8 (inventive)**

492.4 g of HDI and 1.0 g of dibutyl phosphate were heated to 80 °C. Over the course of 1 h a mixture of 12.69 g 1,4-butane diol and 494.9 g of Desmophen L 300 were added dropwise while stirring. The reaction mixture stirred at 80 °C for another 2.5 h until an NCO content of 16.2% was reached. Excess HDI was removed via distillation employing a thin film evaporator at 130 °C and about 0.4 mbar. A turbid prepolymer with an NCO content of 7.2%, a viscosity of 2260 mPas and a residual diisocyanate content of 0.02% was obtained.

**Prepolymer 9 (inventive)**

374.1 g of HDI and 1.0 g of dibutyl phosphate were heated to 80 °C. Over the course of 1 h a mixture of 15.66 g 1,4-butane diol and 610.8 g of Desmophen L 300 were added dropwise while stirring. The reaction mixture stirred at 80 °C for another 2.5 h until an NCO content of 9.4% was reached. Excess HDI was removed via distillation employing a thin film evaporator at 130 °C and about 0.3 mbar. A turbid prepolymer with an NCO content of 5.3%, a viscosity of 16500 mPas and a residual diisocyanate content of 0.03% was obtained.

**Prepolymer 10 (not inventive)**

143 g of HDI and 0.4 g of dibutyl phosphate were heated to 80 °C. Over the course of 1 h a mixture of 2.57 g 1,4-butane diol and 255 g of polyether 1 were added dropwise while stirring. The reaction mixture stirred at 80 °C for another 2.5 h until an NCO content of 11.5% was reached. Excess HDI was removed via distillation employing a thin film evaporator at 130 °C and about 0.7 mbar. A turbid prepolymer with an NCO content of 4.9%, a viscosity of 2560 mPas and a residual diisocyanate content of 0.03% was obtained.

**Prepolymer 11 (not inventive)**

285 g of HDI and 0.7 g of dibutyl phosphate were heated to 80 °C. Over the course of 1 h a mixture of 4.15 g 1,2-ethane diol and 411 g of polyether 1 were added dropwise while stirring. The reaction mixture stirred at 80 °C for another 2.5 h until an NCO content of 14.4% was reached. Excess HDI was removed via distillation employing a thin film evaporator at 130 °C and about 0.7 mbar. A turbid prepolymer with an NCO content of 5.4%, a viscosity of 1850 mPas and a residual diisocyanate content of 0.04% was obtained.

**Prepolymer 12 (not inventive)**

310.2 g of HDI and 0.7 g of dibutyl phosphate were heated to 80 °C. Over the course of 1 h a mixture of 3.90 g 1,12-dodecane diol and 385.9 g of polyether 1 were added dropwise while stirring. The reaction mixture stirred at 80 °C for another 2.5 h until an NCO content of 16.4% was reached. Excess HDI was removed via distillation employing a thin film evaporator at 130 °C and about 0.5 mbar. A turbid prepolymer with an NCO content of 5.2%, a viscosity of 2160 mPas and a residual diisocyanate content of 0.03% was obtained.

**Prepolymer 13 (not inventive)**

316.5 g of HDI and 0.8 g of dibutyl phosphate were heated to 80 °C. Over the course of 1 h a mixture of 12.50 g 1,1,1-Trimethylolpropan and 487.5 g of polyether 1 were added dropwise while stirring. The reaction mixture stirred at 80 °C for another 2.5 h until an NCO content of 12.8% was reached. Excess HDI was removed via distillation employing a thin film evaporator at 130 °C and about 0.7 mbar. A clear prepolymer with an NCO content of 5.6%, a viscosity of 863 mPas and a residual diisocyanate content of 0.02% was obtained.

**Prepolymer 14 (not inventive)**

1680 g of HDI and 5.0 g of dibutyl phosphate were heated to 80 °C. Over the course of 1 h 2960 g of Desmophen L300 were added dropwise while stirring. The reaction mixture stirred at 80 °C for another 3.5 h until an NCO content of 9.1% was reached. Excess HDI was removed via distillation employing a thin film evaporator at 140 °C and about 0.7 mbar. A clear prepolymer with an NCO content of 5.0%, a viscosity of 5140 mPas and a residual diisocyanate content of 0.03% was obtained.

**Prepolymer 15 (not inventive)**

2431 g of HDI and 3.0 g of dibutyl phosphate were heated to 80 °C. Over the course of 1 h 569 g of Desmophen L300 were added dropwise while stirring. The reaction mixture stirred at 80 °C for another 3 h until an NCO content of 35.7% was reached. Excess HDI was removed via distillation employing a thin film evaporator at 140 °C and about 0.6 mbar. A clear prepolymer with an NCO content of 8.8%, a viscosity of 710 mPas and a residual diisocyanate content of 0.02% was obtained.

**Prepolymer 16 (not inventive)**

168 g of HDI and 0.5 g of dibutyl phosphate were heated to 80 °C. Over the course of 1 h 296 g of Desmophen L300 were added dropwise while stirring. The reaction mixture stirred at 80 °C for another 3.5 h until an NCO content of 8.8% was reached. The product had a viscosity of 2100 mPas.

**Prepolymer 17 (not inventive, according to US2006142529, example 4)**

133.5 g of Desmodur 24 MI and 133.5 g Desmodur 44 M were heated to 70 °C. Over the course of 2 h 733.3 g of a Poly(ethyleneglycol-ran-propylenglycol) were added dropwise while stirring. The reaction mixture stirred at 70 °C for another 4 h until a constant NCO content of 5.4% was reached. The product had a viscosity of 29900 mPas.

**Preperation of foams or hydrogels**

The prepolymers from the examples were placed into a 500 mL PP beaker (Sarstedt) and stirred using Disperlux green 037 stirrer at 930 rpm for 15 seconds. Subsequently, a defined amount of aqueous phase (Table 1) was added. Unless stated otherwise the aqueous phase consisted of 93.5% water, 1.3% sodium hydrogen carbonate, 0.4% citric acid monohydrate and 4.8% Pluronic 6800. It was stirred for another 5 seconds. Then the mixture was coated on a release paper (Felix Schoeller Y05200) using a 1500 $\mu$m coating bar. After 40 seconds a pin holed release paper (Felix Schoeller Y05200) was placed on top. The foam or hydrogel was allowed to dry over night at room temperature.

**Table 1. Properties of foams prepared from prepolymer examples.**

| Foam/ Hydrogel example | Prepolymer | Mass ratio prepolymer to aqueous phase | D [g/L] | S | $\sigma_{b,wet}$ [kPa] | $\varepsilon_{b,wet}$ [%] | $\sigma_{b,wet}/$D | $(\sigma_{b,wet}/$D)$\cdot$S$^2$ |
|---|---|---|---|---|---|---|---|---|
| 1 inventive | 1 | 5:1 | 92 | 1.36 | 19 | 320 | 0.21 | 0.38 |
| 2 inventive | 2 | 7:1 | 151 | 1.28 | 58 | 154 | 0.38 | 0.63 |
| 3 inventive | 3 | 7:1 | 87 | 1.28 | 19 | 284 | 0.22 | 0.36 |
| 4 inventive | 4 | 7:1 | 115 | 1.31 | 30 | 226 | 0.26 | 0.45 |
| 5 inventive | 5 | 7:1 | 163 | 1.27 | 75 | 244 | 0.46 | 0.74 |
| 6 inventive | 6 | 7:1 | 92 | 1.31 | 23 | 244 | 0.25 | 0.43 |
| 7 inventive | 7 | 5:1 | 84 | 1.14 | 23 | 658 | 0.27 | 0.36 |
| 8 inventive | 8 | 7:1 | 57 | 1.10 | 30 | 274 | 0.53 | 0.64 |
| 9 inventive | 9 | 7:1 | 111 | 1.23 | 42 | 234 | 0.38 | 0.57 |
| 10 non-inventive | 10 | 5:1 | 351 | 1.47 | 41 | 1113 | 0.12 | 0.25 |
| 11 non-inventive | 11 | 7:1 | 91 | 1.33 | 6 | 470 | 0.07 | 0.12 |
| 12 non-inventive | 12 | 7:1 | 99 | 1.34 | 7 | 461 | 0.07 | 0.13 |
| 13 non-inventive | 13 | 5:1 | 85 | 1.30 | 5 | 34 | 0.06 | 0.10 |
| 14 non-inventive | 14 | 6:1* | 89 | 1.30 | 15 | 300 | 0.17 | 0.28 |
| 15 non-inventive | 15 | 7:1 | 49 | 1.22 | 9 | 67 | 0.18 | 0.27 |
| 16 non-inventive | 16 | 7:1 | 61 | 1.19 | 61 | 235 | 1.00 | 1.42 |
| 17 non-inventive | 17 | 6:1** | 189 | 1.11 | 94 | 50 | 0.50 | 0.61 |

\* The aqueous phase consisted of 91.7% deionized water, 6.5% Pluronic 6800 and 1.8% sodium hydrogencarbonate.
\*\* According to aqueous phase A1 from US2006142529 (98% deionized water and 2% Pluronic 6800).

As already explained in the introduction the term $(\sigma_{B,wet}/$D)$\cdot$S$^2$ is useful to assess the wet-tensile-strength-at-break ($\sigma_{b,wet}$) independent from density and extent of swelling. Table 1 summarizes the properties of foams 1 to 17 prepared from the respective prepolymer examples 1 to 17.

Aliphatic prepolymers containing more than 8% residual diisocyanate like comparative example 16 in this document can be used to produce foams with excellent wet strength performance as indicated by the outstanding $(\sigma_{B,wet}/$D)$\cdot$S$^2$ value of 1.42. Similar prepolymers that differ only by the subsequent removal of the residual diisocyanate monomer are known. For instance, example 1 of WO19137879 leads to a prepolymer that retained a rather low viscosity of 5140 mPas after distillation. However, the $(\sigma_{B,wet}/$D)$\cdot$S$^2$ value of foams produced from this prepolymer dropped drastically to 0.28 (see non-inventive example 14 as described above in table 1). In order to verify that this drop can be attributed to the presence of residual diisocyanate rather than the change in chemical composition of the product accompanying the distillation process (HDI content and polyol content) comparative prepolymer 15 was designed in a way that it resembles the chemical composition of prepolymer 16 (similar HDI and polyol content of 35% and 65%, respectively) but differs in the fact that the residual diisocyanate was removed via distillation. It can be summarized that prepolymers that allow production of foams with good wet-tensile-strength-at-break ($\sigma_{b,wet}$) performance as indicated by high $(\sigma_{B,wet}/$D)$\cdot$S$^2$ values were known in prior art but the reason for the good performance could be attributed to the presence of residual diisocyanates in these disclosed formulations which are often not desirable because of different reasons.

Aliphatic foam 16 and aromatic foam 17 show excellent $(\sigma_{B,wet}/D)\cdot S^2$ values > 0.30 but were prepared from the respective prepolymers 16 and 17 that contain more than 8% monomeric diisocyanates (component A1). Prepolymers 1 to 15 contain less than 8% diisocyanates because residual diisocyanate were removed by distillation. Foams 13 to 15 were prepared from non-inventive respective prepolymers 13 to 15 that do not contain component A3). More specifically, foam 13 was prepared from a non-inventive prepolymer 13 containing a non-inventive trifunctional polyol instead of component A3). Foams 10 to 12 were prepared from non-inventive prepolymers 10 to 12 that contain only 1% of a component A3) in respect to the sum of components A2) to A4). These non-inventive examples 10 to 14 showed $(\sigma_{B,wet}/D)\cdot S^2$ values of $\leq 0.30$.

Surprisingly, foams 1 to 9 that were prepared from inventive prepolymers 1 to 9 that contained less than 8% monomeric diisocyanate and (more than 1% of a component A3) in respect to the sum of components A2) to A4) showed $(\sigma_{B,wet}/D)\cdot S^2$ values $\geq 0.30$. This has been demonstrated for different examples of component A3), specifically 1,2-ethane diol, 1,4-butane diol, 1,6- hexane diol and 1,12-dodecane diol. Also, comparing foams of examples 1 and 4 (prepolymers contained 2.5% diol) with foams of examples 2 and 5 (prepolymers contained 3.5% diol) proved that increased diol concentration led to improved $(\sigma_{B,wet}/D)\cdot S^2$ values of greater 0.60. Additionally, the positive effect of component A3) in concentrations greater 1% with respect to components A2) to A4) was demonstrated for different components A2). Foam examples 10 and 1 show the effect of component A3) in combination with Polyether 1 with OH-number 112 as component A2). Foam examples 14 and 8 show the effect of component A3) in combination with Desmophen L 300 with OH-number 190 as component A2). Foam 6 from prepolymer 6 achieves a good $(\sigma_{B,wet}/D)\cdot S^2$ value by employing Polyol 2 with OH-number 111 as component A2).

**Claims**

1. A process for producing polyurethane foams or polyurethane hydrogels, in which compositions comprising

   A) isocyanate-functional prepolymers obtainable by the reaction of

   A1) low molecular weight diisocyanates of molar mass from 140 to 278 g/mol, with
   A2) polyalkylene oxides having an OH functionality of two or more,
   A3) more than 1% by weight $C_2$ to $C_{12}$ diols based on the total amount of the isocyanate-reactive components A2) to A4) wherein component A3) differs from component A2),
   A4) optionally further isocyanate-reactive components differing from A2) and A3);

   B) water in an amount of at least 2% by weight, based on the total weight of the composition;
   C) optionally polyisocyanates obtainable by reaction of at least two low molecular, preferably aliphatic diisocyanates, wherein the diisocyanates having a molar mass of 140 to 278 g/mol,
   D) optionally catalysts;
   E) optionally salts of weak acids, the corresponding free acids of which have a pKA in water at 25°C of $\geq 3.0$ and $\leq 14.0$;
   F) optionally surfactants; and
   G) optionally mono- or polyhydric alcohols or polyols;
   H) optionally hydrophilic polyisocyanates obtainable by reaction of

   H1) low molecular weight diisocyanates of molar mass from 140 to 278 g/mol and/or polyisocyanates preparable therefrom and having an average isocyanate-functionality of 2 to 6 with
   H2) monohydroxyfunctional polyalkylene oxides of OH number from 10 to 250 and of oxyethylene unit content from 50 to 100 mol%, based on the total amount of the oxyalkylene groups present,

   are provided, optionally foamed and cured wherein the isocyanate containing components, especially components A), C) and H), do not exceed a residual diisocyanate content of 8% by weight, based on the total weight of the polyurethane foam or hydrogel.

2. The process according to claim 1, wherein component A) comprises component A3) within a range from 1.1 to 4.9% by weight, based on the total amount of the isocyanate-reactive components, at least A2) to A4).

3. The process according to either of the preceding claims, wherein the isocyanate-containing components, especially components A), C) and H), have a total isocyanate content within a range from 2% to 12% by weight, based on the total amount of the isocyanate-containing components, especially on the total amount of components A), C) and H).

4. The process according to either of the preceding claims, wherein prepolymer A) has a proportion by weight of low molecular weight diisocyanates having a molar mass of 140 to 278 g/mol of below 1.0% by weight, based on the total mass of the prepolymer A).

5. The process according to either of the preceding claims, wherein aliphatic diisocyanates are used as component A1).

6. The process according to either of the preceding claims, wherein linear diisocyanates are used as component A1).

7. The process according to either of the preceding claims, wherein unbranched diols are used at least partly as component A3).

8. The process according to any of the preceding claims, wherein the isocyanate-functional prepolymer A has a viscosity at RT/ 23°C/ 25°C, determined according to DIN 53019, of ≤ 50 000 mPas.

9. The process according to any of the preceding claims, wherein the following steps are conducted:

   I) preparing the prepolymer A) from components A1), A2), A3) and optionally A4) and optionally D),
   II) optionally mixing components A), C) and H) and other isocyanate-containing components to obtain a prepolymer mixture,
   III) optionally adding A4) and optionally D),
   IV) optionally mixing component B) with all other components, especially D), E), F) and G), apart from the prepolymer mixture,
   V) mixing the prepolymer mixture obtained in I) to III) with the mixture from IV).

10. The process according to any of the preceding claims, wherein the oxyethylene unit content of A2) is ≥ 50% by weight, based on the total amount of the oxyalkylene groups present.

11. The process according to any of the preceding claims, wherein the polyalkylene oxide A2) has an OH number within a range from 25 to 450 mg KOH/g.

12. A polyurethane foam or a polyurethane hydrogel obtained by a process according to any of the preceding claims.

13. A polyurethane prepolymer mixture comprising the following components:

   A) a polyurethane prepolymer obtainable by the reaction of

      A1) low molecular weight diisocyanates of molar mass from 140 to 278 g/mol, with
      A2) polyalkylene oxides having an OH functionality of two or more,
      A3) more than 1% by weight of $C_2$ to $C_{12}$ diols based on the total amount of the isocyanate-reactive components A2) to A4) wherein component A3) differs from component A2),
      A4) optionally further isocyanate-reactive components differing from A2) or A3),

   C) optionally polyisocyanates obtainable by reaction of at least two low molecular, preferably aliphatic diisocyanates, wherein the diisocyanates having a molar mass of 140 to 278 g/mol,
   D) optionally catalysts;
   H) optionally hydrophilic polyisocyanates obtainable by reaction of

      H1) low molecular weight diisocyanates of molar mass from 140 to 278 g/mol and/or polyisocyanates preparable therefrom and having an average isocyanate functionality of 2 to 6 with
      H2) monohydroxyfunctional polyalkylene oxides of OH number from 10 to 250 and of oxyethylene unit content from 50 to 100 mol%, based on the total amount of the oxyalkylene groups present,

   wherein the polyurethane prepolymer mixture, especially components A), C) and H), has an isocyanate content within a range from 2% to 8% by weight and a content of urethane groups of 1.0 to 3.5 mol/kg, based in each case on the total amount of the polyurethane prepolymer mixture.

14. The use of a polyurethane prepolymer mixture according to claim 13 or of a polyurethane foam or hydrogel according to claim 12 for production of a wound dressing, a cosmetic article or an incontinence product.

15. A wound dressing, a cosmetic article or an incontinence product obtainable using polyurethane foams or hydrogels according to any of claims 12 or 13, or produced according to any of claims 1 to 11.

Europäisches
Patentamt

European
Patent Office

Office européen
des brevets

# EUROPEAN SEARCH REPORT

Application Number

EP 20 20 6269

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2019/137879 A1 (COVESTRO DEUTSCHLAND AG [DE]) 18 July 2019 (2019-07-18) <br> * examples 1-10; table 1 * <br> * page 7, line 1 - line 9 * <br> ----- | 1-15 | INV. <br> C08G18/32 <br> C08G18/48 <br> C08G18/73 <br> C08G18/30 |
| A,D | US 2006/142529 A1 (THIEDE VERENA [DE] ET AL) 29 June 2006 (2006-06-29) <br> * examples 1-11 * <br> ----- | 1-15 | A61F13/00 <br> A61L15/60 <br> C08G18/16 |

**TECHNICAL FIELDS SEARCHED (IPC)**

C08G
A61F
A61L

The present search report has been drawn up for all claims

| Place of search | Date of completion of the search | Examiner |
|---|---|---|
| Munich | 9 April 2021 | Sütterlin, Martin |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

........................................................................

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 20 20 6269

This annex lists the patent family members relating to the patent documents cited in the above-mentioned European search report.
The members are as contained in the European Patent Office EDP file on
The European Patent Office is in no way liable for these particulars which are merely given for the purpose of information.

09-04-2021

| Patent document cited in search report | | Publication date | Patent family member(s) | | Publication date |
|---|---|---|---|---|---|
| WO 2019137879 | A1 | 18-07-2019 | US | 2019218329 A1 | 18-07-2019 |
| | | | WO | 2019137879 A1 | 18-07-2019 |
| US 2006142529 | A1 | 29-06-2006 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2006142529 A
- US 3903232 A
- US 4137200 A
- US 5849850 A
- EP 2143744 A
- EP 2470580 A
- EP 2632501 A
- WO 19137879 A
- WO 2011006608 A
- EP 17156493
- WO 0180994 A

**Non-patent literature cited in the description**

- Ullmanns Encyclopädie der technischen Chemie [Ullmann's Encyclopedia of Industrial Chemistry. Verlag Chemie, vol. 19, 31-38